# EUROPEAN PATENT SPECIFICATION

(11) **EP 2 516 619 B1**
(45) Date of publication and mention of the grant of the patent: **25.01.2017**
(21) Application number: 10839905.6
(22) Date of filing: 22.12.2010
(51) Int. Cl.: C12M 3/06, B01D 37/04, C12M 1/02, C12N 5/09, B01D 35/20

(54) **DEVICE AND METHOD FOR BIOLOGICAL SAMPLE PURIFICATION AND ENRICHMENT**
VORRICHTUNG UND VERFAHREN ZUR REINIGUNG UND ANREICHERUNG EINER BIOLOGISCHEN PROBE
DISPOSITIF ET PROCÉDÉ POUR LA PURIFICATION ET L'ENRICHISSEMENT D'ÉCHANTILLON BIOLOGIQUE

(30) Priority: 22.12.2009 SE 0951009
(43) Date of publication of application: 31.10.2012
(73) Proprietor: Icellate Medical AB, 114 37 Stockholm (SE)
(72) Inventor: Ericsson, Christer, 128 67 Sköndal (SE); Castro, Juan, 178 93 Drottningholm (SE); Wännmar, Lars, 117 41 Stockholm (SE)
(74) Representative: Karlström, Lennart
(86) International application number: PCT/SE2010/051473
(87) International publication number: WO 2011/078784

(56) References cited:
- EP-A1- 0 987 325
- EP-A1- 1 683 857
- WO-A1-92/22373
- WO-A1-2008/107652
- WO-A2-03/053346
- JP-A- 2009 284 860
- US-A1- 2003 134 416
- US-A1- 2005 025 755

## Description

### Field of the Invention

The invention relates to the field of biological sample purification. More specifically, the invention relates methods which can separate trace target cells from a fluid sample having a plurality of other cells and/or contaminants.

### Background of the Invention

Efficient and effective separation of various components in a biological sample is essential for many areas of research and medicine. Many liquid samples comprise a plurality of cells and/or contaminants of little or no interest, and very few cells of interest for further evaluation. Thus, being able to separate large numbers of normally-occurring cells such as blood or epithelial cells from trace numbers of target cells is desired.

A number of technologies are available in the art to address this need. For example, it is known to pass a fluid sample through a filter thus purifying certain components of the sample. US 7,494,809 B2 discloses an automated process in which a fluid biological sample is passed through a series of filters and treated with reagents in order to provide a stained, enriched cell population for evaluation.

One cell population which can be of interest is circulating tumour cells or CTC. This because the greatest risk for mortality among cancer patients is often not the original malignant tumour, but the formation of metastases in tissues or organs distant from the primary tumour. Small numbers of cells can break free from the primary tumour and enter the circulatory system, becoming CTC. While the majority do not survive, some not only survive, but at some point translocate across the endothelial wall, establishing a new tumour in the surrounding tissue.

CTC detection has largely depended upon antibody-based positive selection.

However, this technique is limited to available antibodies against known tumour cell biomarkers. One way to address this shortcoming is filtration technology.

Filters particularly suited to separating circulating tumour cells from a sample have been described, see for example Published Patent Application US 2006/0254972 A1.

JP 2009 284860 discloses an apparatus for concentration of mononuclear cells and platelets and does not concern tumour cells, and no pressure measurer is disclosed.

US2005/025755 and WO 03/053346 are concerned with preparing adipocytes for use in autologous cell transfer of cell tissue and does not mention that a pressure sensor is used to stop filtration when a certain pressure is reached.

WO 2008/107652 mentions filtration of cancer cells from blood but does not discuss how performance can be improved by stopping filtration when a predetermined pressure is reached.

However, despite the available techniques, there remains a need in the art for devices and methods which offer efficient and effective purification and enrichment of circulating tumour cells in a liquid biological sample.

### Summary of Invention

Therefore, it is an object of the present invention to provide methods which offer efficient and effective purification and enrichment of components in a liquid biological sample.

As noted above, by using present technologies one may be faced with one or more problems in evaluating a sample, including low target cell density, contamination, and a need for specialised training or equipment to observe non-native cells. Furthermore, the shortcomings of existing technologies can require expensive and time consuming, possibly painful, re-sampling. Delays in diagnosis can affect patient outcome, as prompt and precise detection of CTCs with metastatic potential can drastically increase survival rates.

Although removing CTC from the bloodstream of a patient can be of use, it is of far greater benefit if the removed cells are in sufficiently good condition to be analysed. This analysis not only confirms the target cells were present in the sample, it may be useful in other ways, e.g. to help predict the potential course of a disease.

The present invention meets this need where the present technology fails by not only separating target cells from a plurality of other cells and/or contaminants in a liquid sample, but by providing the purified, enriched target cell population in a relatively native state.

Yet another benefit of the present invention may be the provision of faster diagnostic information. Because the invention allows for a rapid separation of target cells, and offers those cells in a native state, the time from sample-taking to target cell analysis is minimised. The effectiveness of the invention can also reduce the need for expensive and time-consuming repeat sampling.

Via a physiological process including a series of liquid transfer steps through an agitating filter, the target cells are effectively and efficiently removed from the greater sample and provided in a native state. A further benefit of the present invention is that the non-target components of the sample are also preserved in their native state. In many cases these components are also of interest. A method which allows for observation and analysis these components as well is therefore an even greater advance in the art.

It is disclosed but not part of the invention, a device for sample purification and enrichment comprises a filtration unit having a first side and a second side and comprising an agitator, a sample input in elective fluid communication with the first side of the filtration unit, a first liquid reservoir in elective fluid communication with the first side of the filtration unit, a first suction means having a first suction reservoir and in elective fluid communication with the first side of the filtration unit, a second liquid reservoir in elective fluid communication with the second side of the filtration unit, a pressure measurer in fluid communication with the second side of the filtration unit and having a flow stop ability, and a second suction means in elective fluid communication with the second side of the filtration unit.

The second suction means can have a second suction reservoir. At least one of the first suction means and the second suction means can be a peristaltic pump. The pressure measurer flow stop ability can involve a clamp or a piezo-electric membrane.

A cartridge for use in the inventive device is provided which comprises a filtration unit having a first side and a second side and comprising a filter and being connectable to an agitator, a sample input tube in fluid communication with the first side of the filtration unit, a first liquid reservoir tube in fluid communication with the first side of the filtration unit, a first suction reservoir tube in fluid communication with the first side of the filtration unit, a second liquid reservoir tube in fluid communication with the second side of the filtration unit, and a second suction tube in fluid communication with the second side of the filtration unit, wherein the cartridge has at least one access point for a pressure measurer.

The filtration unit can comprise an agitator.

According to an embodiment of the present invention, a method of purifying a sample is provided which comprises the steps of providing a sample to a sample input in fluid communication with a first side of a filter, applying suction to a second side of a filter to draw fluid from the sample input through the filter until a predetermined pressure is measured, placing the second side of the filter in exclusive fluid communication with a reservoir containing a liquid, applying suction to the first side of the filter to draw fluid from the reservoir through the filter, and collecting the fluid drawn from the reservoir through the filter in a container. The filter is subject to agitation during at least a significant portion of the process and the purified sample is collected in the container.

In such a method the pre-determined pressure can be in the range of approximately 130-150, preferably 135-145, even more preferably 140 mm H₂0 when measured using a water column.

While the benefits of the invention described herein often cease by noting the purified and enriched target cells can be analysed for morphology, it will be obvious to a skilled person that there are a number of downstream applications which are relevant to this technology. For example, assessing metastatic potential, screening high-risk populations, diagnosing disease, predicting disease, predicting treatment outcome including assessing drug sensitivity, monitoring a disease state, monitoring response to therapy, optimizing treatment regimens, and developing new therapies, to name a few.

Unless otherwise noted, all technical and scientific terms used herein have the same meaning as commonly understood by a skilled person. The equipment and methods referred to generally are well-known and commonly employed in the art.

A sample can be blood from any source, such as an organism. A sample can include cord blood, or peripheral blood. It may comprise additives such as anticoagulants or stabilisers.

A target cell is a specific type of cell, namely circulating tumour cells (CTCs) of interest which may or may not be present in the sample. Non-target cells are those which are preferably removed, even though their analysis may be of interest.

A "filter" is a structure that comprises one or more openings or pores of a particular dimension which allows the passage of particles smaller than that dimension to an opposite side of the filter while preventing larger particles from passing through the filter. It may be formed of any material, for example, plastics, ceramics, silicon, metal, or glass. The filter may be microfabricated or micromachined, alternatively, it may be a more conventional filter such as mono- or multi-fibrous filters made of nylon, polycarbonate, celluloses, etc. A filter may be treated to alter its surface properties, such as to increase hydrophilicity.

Herein, components and materials used in relation to the invention are often described, explicitly or implicitly, as disposable. A skilled person will be aware of the demands for sterility and decontamination for devices and methods of this sort, which often weigh in favour of disposable materials. Furthermore, the time and materials required for sterilisation and documentation of the same, and the risk for error, generally make that route less advantageous. However, this should not in any way be taken to preclude the use of re-useable materials when desired and appropriate.

### Brief Description of the Drawings

Various aspects of the invention will be described by way of example with reference to the accompanying drawings, in which:
Figure 1 is a schematic view of a device;
Figure 2 is a schematic view of a further aspect f a device;
Figure 3 shows an untreated bladder washing sample;
Figure 4 shows the sample of Figure 3 after treatment according to the invention;
Figure 5 shows an untreated ascites sample; and
Figure 6 shows the sample of Figure 5 after treatment according to the invention.

### Detailed Description

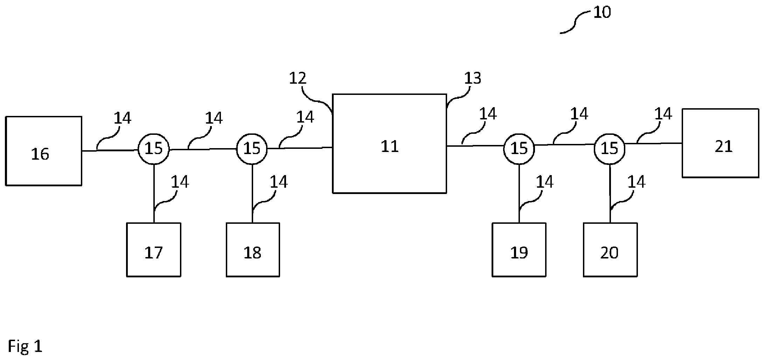

As described above, the invention relates to a method for sample purification and enrichment. Referring by way of example to Figure 1, a device 10 comprises a filtration unit 11 having a first side 12 and a second side 13 and comprising an agitator (not shown). The first and second sides of the filter 12, 13 are generally opposing, but not necessarily strictly opposite each other. Depending on the configuration and placement of the actual filtration material, the filter, their relative positions may be closer together or further apart.

Filtration unit 11 comprises a filter (not shown) which may be any suitable filter as known in the art. The pore size of the filter should be selected based on a consideration of both the target cells of interest and the predicted non-target cells and/or contaminants expected to be in the sample. For example, when using a device 10 of the invention to purify and enrich CTCs from samples including blood cells, a filter size in the range of 10pm is preferred. The size of the filter itself can vary depending on the device and the purpose. In an embodiment of the invention a standard commercial 22 mm diameter filter is used, and is capable of filtering about 15,000 - 20,000 cells.

The agitator which is provided in conjunction with filtration unit 11 allows mechanical vibration to be used during filtration to maximise efficiency and precision of the device 10. Mechanical agitation in any direction (relative to the overall orientation of the device) may be effective, however, agitation in a horizontal direction has been found to work particularly well with biological samples in a device 10 of the invention. For example, agitating a filtration unit which contains some headspace for gases in a back and forth horizontal motion can result in the fluid within the filtration unit swirling in a circumferential motion. This motion appears to be effective while at the same time gentle. Agitation in the range of 1000-2000 Hz may be preferred. This range generally allows for effective use of the entire surface area of the filter while not being so strong as to damage or cause rupture to components in the sample.

In a device 10 the various components are in fluid communication with one another via a series of tubes 14. The broad range of uses of the device is accompanied by a significant degree of choice in commercially-available tubing. It will be preferred to employ materials which are inert with respect to biological fluids and components thereof. Furthermore, the length and diameter, particularly the inner diameter, of the tubing should be taken into consideration when determining system parameters such as speed and pressure. In some cases it may be preferred to use silicone tubing with an internal diameter in the range of 2 mm.

While described herein as generally flexible cylindrical forms, tubes 14 of the invention can comprise non-flexible and/or non-cylindrical materials. For example, glass or metal formed into cylinders or other shapes. The tubing serves the purpose of conveying liquids from and between various components of the device and as such a great deal of variety of forms may be used depending on the chosen dimensions and configuration of the overall unit. Skilled persons will appreciate that the inner dimensions of the tubes need be sufficiently large to accommodate the cells in the sample, knowing that diameters which approach the size of the cells may cause pulsing or other undesirable effects on fluid flow within the device.

A plurality of selection means 15 are provided which allow selective fluid communication between various components of the device. Selection means can direct and stop fluid passage via manual or automated control. For example, a two-way valve can be used which could be adjusted into a first position to put a first component into fluid communication with the filter, then rotated into a second position putting a second component into fluid communication with the filter. At different areas of the device 10 selection means 15 may differ, for example some may be manual and some may be automated.

A number of components are located in fluid communication with the first side of the filter 12, including a sample input 16, a first liquid reservoir 17, and a first suction means 18.

Sample input 16 can be a port or other means to allow a fluid sample access to the system. Alternatively, it may comprise a holder or container, such as a test tube. In its simplest form it is merely an extension of a tube 4 which can be placed in communication with a sample, which could be provided in, for example, a test tube. Due to concerns regarding, i.e., contamination and security, the contents of the sample may preferably be enclosed, for example by providing the sample in a stoppered test tube and configuring the sample input as a needle at the end of a tube, which needle is inserted into the stopper.

First liquid reservoir 17 is provided to the device 10. In initial stages of use a liquid is present in first liquid reservoir 17. Its use will be further described in the examples, below, however it can be noted that the fluid in first liquid reservoir serves at least a general flushing or rinsing purpose and as such is preferable physiologically neutral. Suitable fluids include phosphate buffered saline (PBS), Hank's buffered saline solution, (hydroxymethyl)aminomethane (TRIS) buffer, and other buffers with a physiologically neutral pH and osmolarity of approximately 270-330 mOsm/kg.

First suction means 18 is capable of providing suction to a first side of the filter 12, effectively drawing fluid from the second side of the filter 13 to the first side of the filter 12. First suction means 18 is provided with a first suction reservoir (not shown) which is capable of collecting fluid and particles suspended therein. In its simplest form first suction means 18 may be a syringe the stopper of which can be drawn out to create suction and the inner cavity of which provides a reservoir. Alternatively, the first suction means 8 can be a peristaltic pump.

There are also a number of components located in fluid communication with the second side of the filter 13, including a second liquid reservoir 19, a pressure measurer 20, and a second suction means 21.

Second liquid reservoir 19 is initially supplied to device 10 with fluid therein. Use will be further described below, however, the fluid is preferably physiologically neutral and as a consequence the material used to form second liquid reservoir 19 is preferably non-reactive. The size and/or capacity of second liquid reservoir 19 will depend on the intended use of device 10 and will also depend on whether second liquid reservoir 9 is adapted as a fixed portion or if it is interchanged after every use of device 10.

Pressure measurer 20 allows a user (or automated monitoring device) to monitor the degree of vacuum present within the device. Through calibration, this measure will allow a determination of when the filter is sufficiently saturated with target cells. Pressure measurer 20 may include a visual display.

Pressure measurer 20 further allows a user to normalize a sample. By relying on pressure of the internal system one can avoid the need for timely pre-screening steps which would otherwise be beneficial for determining the concentration of particulate matter in the sample. As the samples and organisms from which they originate will differ, so too will the samples evaluated. Furthermore, users may in some cases choose to dilute a sample so that it can be used for a number of purposes, or to make the purification and enrichment method function most efficiently.

An example of a suitable pressure measurer 20 is a column of water. When such a pressure measurer 20 is employed, a reasonable operating range for the device 10 is 130-150 mm H₂0. The range of 135-145 mm H₂0 may be preferred.

Approximately 140 mm H₂0 may be particularly effective.

Where the device 10 is at least partially automated, pressure measurer 20 is connected to a selection means 15 or other flow control means such that, upon reaching certain predetermined pressures, the aspects of the system are engaged or disengaged.

Particularly where automation is desired, pressure measurer 20 need not include a display means as is inherent in a water column. Instead, it may comprise an electronic sensor which detects pressure. The information obtained by pressure measurer 20 could be transmitted to a central control unit, which could send commands which, using the flow stop ability of the pressure measurer 20, stops or starts flow in chosen areas of the device.

Flow stop ability could be achieved using, for example, a membrane. One example is a piezo-electric membrane. Simple flow stops are also contemplated, such as a clamp or crimp which can physically deform a flexible tube when engaged, thus stopping flow through the tube. By disengaging the clamp or crimp flow may begin again.

Where a physical device is used to achieve the flow stop ability of pressure measurer 20, it need not necessarily be in precisely the same location of the device as the pressure measurer 20.

Pressure measurer 20 can be a device which directly measures pressure; alternatively it may be capable of collecting information which can be used to calculate pressure.

While pressure measurer 20 serves a purpose when conducting the initial pass of the sample through the filtration unit 11 , in subsequent steps using the device 10 it may not be required. Nonetheless, particularly in automated systems, one can continue to measure the internal system pressure for statistical purposes and/or as a safety check to possibly detect malfunctions in the system.

Second suction means 21 is capable of providing suction to a second side of the filter 13, effectively drawing fluid from the first side of the filter 12 to the second side of the filter 13. An optional second suction reservoir may be provided (not shown) which could be capable of collecting fluid and particles suspended therein.

A second suction means 21 could be a syringe the stopper of which can be drawn out to create suction. The inner cavity of the syringe could serve as a reservoir if desired. Alternatively second suction means 21 could be a pump, such as a peristaltic pump. The use of device 10 will be further described below, however it can be noted that, should the non-target cells be of interest a second suction reservoir is preferably provided to conveniently store such cells.

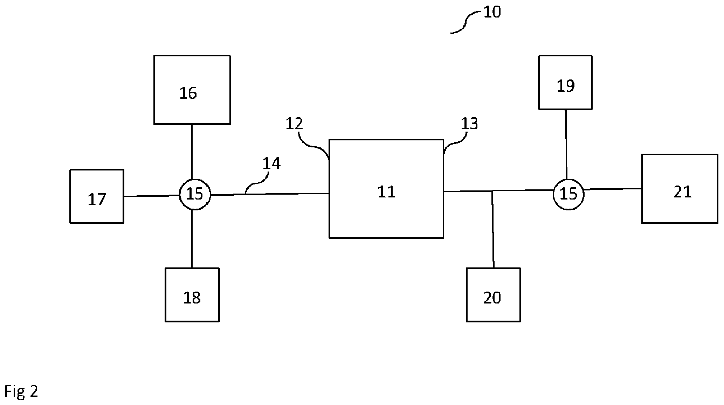

A further embodiment of a device 10 of the invention is shown in Figure 2. In that figure, for ease of illustration, not every piece of tubing 14 is marked with a reference numeral.

In the Figure 2 embodiment a plurality of selection means 15 are shown. The selection means 15 in fluid communication with the first side of the filter 12 is in this embodiment a three-way valve, whereas that in fluid communication with the second side of the filter 13 is a two-way valve.

The embodiment of Figure 2 has an alternative configuration of elements, grouping all components connected to the first side of the filter 12 via a single selection means 15 and tube 14. The components connected to the second side of the filter 13 are also in a different configuration relative to the Figure 1
embodiment; here pressure measurer 20 is not connected via a selection means but instead one of second liquid reservoir or second suction means 21 may be in fluid communication with the system at any time.

It should be apparent to skilled persons that further configurations are possible and within the scope of the invention. Furthermore, a plurality of devices can be provided in sequence or in parallel.

In cases where it is preferred or even essential that the sample is maintained at low operating temperatures, for example, where it is highly desirable to stop cellular activity for the benefit of further analysis or activities, an entire device according to the invention can be provided in a refrigerated unit or cold room. In other cases, it may suffice that the liquid reagents used within the system are provided at a particular temperature, for example, between about 0-4°C.

As previously mentioned it is contemplated that most or all components of a device disclosed herein may be disposable. In particular, it is contemplated that disposable cassettes or cartridges may be supplied with components of a device. Such a cassette can preferably include a barcode or space to adhere a barcode to help facilitate electronic processing of samples. Another option is electronic identifiers which can be incorporated into or affixed to the cassette.

Cassettes or cartridges useful with the present invention comprise a filtration unit and may comprise an agitator. Alternatively, the filtration unit may simply be capable of being connected or engaged by an agitator. A plurality of tubes are also provided which connect respective first and second sides of the filtration unit to the reservoirs and inputs of the device. At least an access point is provided for a pressure measurer, the pressure measurer itself may be included in the cassette. The access point could simply be a place in the tubing where a sensor is inserted.

A device disclosed herein may be operated manually; alternatively it may be partially or entirely automated. For ease of reference Figure 1 is referred to herein.

If desired, the system and/or filter can be primed with a liquid. A sample is provided to a sample input 16 which is in fluid communication with the filtration unit 11. Optionally, the sample may be pre-treated prior to performing the method of the invention. For example, to decrease the propensity of cells to flex and thus be able to pass through a pore smaller than their general diameter, they can be fixed.

When analysing blood samples, the plasma can be separated from the cells treated with a standard 0.37% formaldehyde solution. PBS or other neutral liquid can then be added up to a volume of the original sample. While not wanting to be bound by theory, it is believed that the cells may be fixed or more rigid after the formaldehyde addition, making them pass or fail to pass through the filter in a manner more closely related to their diameter, and less closely related to their ability to flex through a filter pore.

The relevant selection means 15 is configured so that second suction means 21 is in fluid communication with the filtration unit 11. Second suction means 21 is engaged thus drawing fluid and suspended particles via sample input 6 into filtration unit 11.

A range of suitable pressures can be used, depending on the type of sample and configuration of the device. Through the use of pressure measurer 20 it is possible to calculate approximately how saturated the filtration unit 11 has become with target cells. At the appropriate time, for example, when a column of water used to indicate pressure indicates approximately 140 mm H2O, second suction means 21 ceases to apply suction and sample input 16 is taken out of fluid communication with filtration unit 11.

This step has the effect of drawing non-target cells and contaminants smaller than the pore size of the filter through the filter, leaving target cells on the first side of the filtration unit.

Another optional pre-treatment would be to use known materials and methods to achieve selective cellular lysis. This may allow for a faster or more efficient filtration using the device of the invention.

First liquid reservoir 17 is placed in fluid communication with filtration unit 11 and second suction means 21 is again engaged, drawing fluid from first liquid reservoir 17 through filtration unit 11.

This step has the effect of rinsing or cleaning the system of remaining non-target cells. First liquid reservoir 17 is removed from fluid communication with the filtration unit and second liquid reservoir 19 is put into communication with the system. First suction means 18 is engaged, drawing fluid out of second liquid reservoir 19 and through filtration unit 11. The fluid thus drawn is collected in a first suction reservoir (not shown).

Partly due to the flow of fluid and enhanced by the vibration of the filter, target cells which were left on the first side of filter 12 are suspended in the passing fluid and carried into the first suction reservoir. From that point they may undergo direct observation or analysis, treatment or staining, or transfer to another device or storage.

Preferably, second suction means 21 is again used to draw fluid from first fluid reservoir 17 through filtration unit 11 , followed by first suction means 8 drawing additional fluid from second fluid reservoir 19. Even more preferably the immediately aforementioned dual fluid transfer step is repeated twice or three or more times. These exchanges help to ensure particles in the sample are completely removed from the filter and help clean the internal components of device 0.

An analysis done with an embodiment of device 10 according to the invention which included a second suction reservoir has shown that at least 96% of the cells in a sample were captured in either the first or second suction reservoir, demonstrating the outstanding efficiency of the device.

### Example One Bladder Washing Contaminated with Red Blood Cells

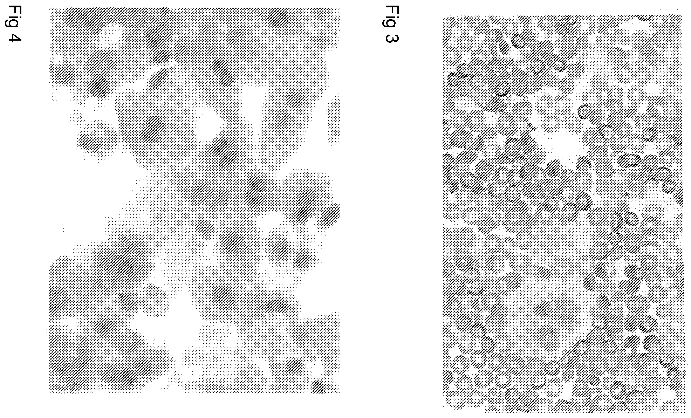

A bladder washing is performed on an organism and the washing fluid, with probable suspended cells and contaminants is collected. A preliminary microscopic analysis on a stained droplet of the sample confirms the presence of prolific numbers of red blood cells and small numbers of potential target cells, see Figure 3. The remaining sample is provided to the sample input of a device of the invention and filtered.

Process time is about 10 minutes. The maximum pressure reached in the device during the first suction of the sample through the filter is 140 mm H₂O. The material captured in the first suction reservoir is placed on a slide and stained. Upon microscopic inspection, the purified and enriched target cells are readily visible in their natural state, see Figure 4.

For control purposes, a second suction reservoir is used. The material captured in the second suction reservoir is placed on a slide and stained. Upon microscopic inspection, it reveals a plurality of red blood cells and epithelial cells. Based on analysis of the target and non-target cells captured, the recovery yield of target cells is calculated to be >95%.

### Example Two Ascites Fluid Contaminated with Epithelial Cells

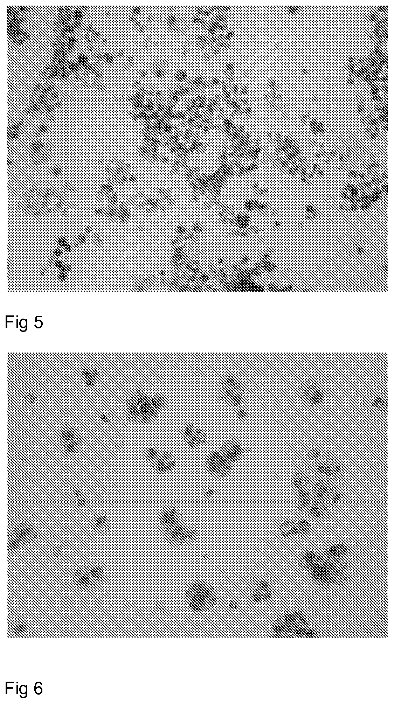

A sample of ascites fluid is obtained. A stained droplet of the fluid is examined microscopically, showing significant amounts of epithelial cells and small numbers of potential target cells, see Figure 5. The 10 ml remaining sample was placed in a 200 ml sample input vessel and filtered through a device of the present invention.

The process was concluded in approximately 10 minutes, during which the maximum pressure reached during the first suction of the sample through the filter was 140 mm H₂O. The material captured in the first suction reservoir was placed on a slide and stained. Upon microscopic inspection, the purified and enriched target cells were readily visible in their natural state, see Figure 6.

### Example Three Dual Step Purification and Enrichment

A sample containing cells, including peripheral blood cells, is provided. The cells are chemically lysed according to known techniques. The cell contents are placed in a sample input vessel of a device according to the invention.

A first process is carried out using a filter whose pores allow free passage to most subcellular components, but retain the largest: cell nuclei. After this run of the system the nuclei are set aside for later use and the remaining components collected in the second suction reservoir are removed. The system is primed again for use and the components previously collected in the second suction reservoir are loaded in the sample input.

A second process is carried out with a filter provided with pores which allow free passage to most cellular components, but retain the large mitochondria from this fraction. Subsequent to this process the nuclei, mitochondria, and the remaining components collected in the second suction reservoir are separately analysed. This analysis confirms that the inventive device can be used with a multi-stage separation strategy to obtain differently-sized components of a fluid sample in a fast and efficient way, in this case subcellular components of a lysed cell. The components, thus separated, were in their native state and largely undisturbed, allowing for further analysis as desired.

The foregoing description and examples have been set forth merely to illustrate the invention and are not intended to be limiting. Since modifications of the described embodiments incorporating the spirit and substance of the invention may occur to skilled persons, the invention should be construed broadly to include all variations within the scope of the appended claims and equivalents thereof.

## Claims

1. A method of purifying a biological sample comprising circulating tumour cells, comprising the steps of:
providing a sample to a sample input in fluid communication with a first side of a filter;
applying suction to a second side of a filter to draw fluid from the sample input through the filter until a pre-determined pressure of approximately 130-150 mm H₂O when measured using a water column is measured with a pressure measurer in fluid communication with the second side of the filter, said pressure measurer having a flow stop ability;
stopping the flow using the flow stop ability once the predetermined pressure is attained
placing the second side of the filter in exclusive fluid communication with a reservoir containing a liquid;
applying suction to the first side of the filter to draw fluid from the reservoir through the filter; and
collecting the fluid drawn from the reservoir through the filter in a container;
wherein the filter is subject to agitation during filtration,
wherein the purified sample is collected in the container.

2. A method according to claim 1 wherein the pre-determined pressure is in the range of approximately 135-145 mm H₂O when measured using a water column.

3. A method according to any one of claims 1or 2 wherein the agitation is mechanical vibration in a horizontal direction.

4. A method according to any one of claims 1 to 3 wherein the collecting step is followed by at least once of a dual fluid transfer step of:
placing the first side of the filter in exclusive fluid communication with a first fluid reservoir containing a liquid;
applying suction to the second side of a filter to draw fluid from the first fluid reservoir through the filter;
placing the second side of the filter in exclusive fluid communication with a second reservoir containing a liquid;
applying suction to the first side of the filter to draw fluid from the second reservoir through the filter; and
collecting the fluid drawn from the second reservoir through the filter in a container.

## Patentansprüche

1. Verfahren zum Reinigen einer biologischen Probe, umfassend Zirkulation von Tumorzellen, umfassend der folgenden Schritte:
bereitstellen einer Probe zu einer Probeneingabe in Flüssigkeitsverbindung mit einer ersten Seite eines Filters;
ansaugen auf einer zweiten Seite eines Filters, um Flüssigkeit aus dem Probeneingang durch den Filter zu saugen, bis mit einem Druckmesser ein vorgegebener Druck von etwa 130 - 150 mm H₂O gemessen werden, unter Verwendung einer Wassersäule, welcher in Flüssigkeitsverbindung mit der zweiten Seite des Filters steht, wobei der Druckmesser eine Strömungsstoppfähigkeit aufweist;
stoppen der Strömung unter Verwendung der Strömungsstoppfähigkeit, sobald der vorgegebene Druck erreicht wird;
anordnen der zweiten Seite des Filters in einer exklusiven Flüssigkeitsverbindung mit einem Behälter, der eine Flüssigkeit enthält;
ansaugen der ersten Seite des Filters, um Flüssigkeit aus dem Behälter durch den Filter zu saugen; und
sammeln der aus dem Behälter gesaugten Flüssigkeit durch den Filter in einem Behälter;
wobei das Filter während der Filtration umgerührt wird,
wobei die gereinigte Probe im Behälter angesammelt wird.

2. Verfahren nach Anspruch 1, wobei der vorgegebene Druck, der mit einer Wassersäule gemessen wird, im Bereich von etwa 135 - 145 mm H₂O liegt.

3. Verfahren nach einem der genannten Ansprüche 1 oder 2, wobei das Umrühren eine mechanische Vibration in einer horizontalen Richtung entspricht.

4. Verfahren nach einem der genannten Ansprüche 1 bis 3, wobei der Sammelschritt mindestens einmal auf einen Zweiflüssigkeitsübertragungsschritt folgt:
anordnen der ersten Seite des Filters in einer exklusiven Flüssigkeitsverbindung mit einem ersten Flüssigkeitsbehälter, das eine Flüssigkeit enthält;
ansaugen auf die zweite Seite eines Filters, um Flüssigkeit vom ersten Flüssigkeitsbehälter durch das Filter zu saugen;
anordnen der zweiten Seite des Filters in einer exklusiven Flüssigkeitsverbindung mit einem zweiten Behälter, der eine Flüssigkeit enthält;
ansaugen der ersten Seite des Filters, um Flüssigkeit aus dem zweiten Behälter durch das Filter zu saugen; und
ansammeln der Flüssigkeit, das aus dem zweiten Behälter durch das Filter in einem Behälter gesaugt wurde.

## Revendications

1. Procédé de purification d'un échantillon biologique comprenant des cellules tumorales circulantes, comprenant les étapes de :
adresser un échantillon à une entrée d'échantillon en communication fluidique avec un premier côté d'un filtre ;
appliquer une succion à un second côté d'un filtre pour aspirer du fluide à partir de l'entrée d'échantillon à travers le filtre jusqu'à ce qu'une pression prédéterminée d'approximativement 130-150 mm H₂O lorsqu'elle est mesurée à l'aide d'une colonne d'eau soit mesurée avec un mesureur de pression en communication fluidique avec le second côté du filtre, ledit mesureur de pression ayant une capacité d'arrêt de l'écoulement ;
arrêter l'écoulement à l'aide de la capacité d'arrêt de l'écoulement une fois que la pression prédéterminée est atteinte
placer le second côté du filtre en communication fluidique exclusive avec un réservoir contenant un liquide ;
appliquer une succion sur le premier côté du filtre pour aspirer du fluide à partir du réservoir à travers le filtre ; et
collecter le fluide aspiré à partir du réservoir à travers le filtre dans un conteneur ;
dans lequel le filtre est soumis à une agitation pendant la filtration,
dans lequel l'échantillon purifié est collecté dans le conteneur.

2. Procédé selon la revendication 1, dans lequel la pression prédéterminée se situe dans la plage d'approximativement 135-145 mm H₂O lorsqu'elle est mesurée à l'aide d'une colonne d'eau.

3. Procédé selon l'une quelconque des revendications 1 ou 2, dans lequel l'agitation est une vibration mécanique dans une direction horizontale.

4. Procédé selon l'une quelconque des revendications 1 à 3, dans lequel l'étape de collecte est suivie par au moins une fois d'une étape de transfert de fluide double de :
placer le premier côté du filtre en communication fluidique exclusive avec un premier réservoir de fluide contenant un liquide ;
appliquer une succion sur le second côté d'un filtre pour aspirer du fluide à partir du premier réservoir de fluide à travers le filtre ;
placer le second côté du filtre en communication fluidique exclusive avec un second réservoir contenant un liquide ;
appliquer une succion sur le premier côté du filtre pour aspirer du fluide à partir du second réservoir à travers le filtre ; et
collecter le fluide aspiré à partir du second réservoir à travers le filtre dans un conteneur.
